(19) Europäisches Patentamt
European Patent Office
Office européen des brevets

(11) Veröffentlichungsnummer: **0 563 747 B1**

(12) # EUROPÄISCHE PATENTSCHRIFT

(45) Veröffentlichungstag der Patentschrift: **30.08.95**

(51) Int. Cl.[6]: **C07C 227/08**, C07C 231/12

(21) Anmeldenummer: **93104725.2**

(22) Anmeldetag: **23.03.93**

(54) **Verfahren zur Herstellung von wässrigen Betainlösungen.**

(30) Priorität: **03.04.92 DE 4211190**

(43) Veröffentlichungstag der Anmeldung:
**06.10.93 Patentblatt 93/40**

(45) Bekanntmachung des Hinweises auf die Patenterteilung:
**30.08.95 Patentblatt 95/35**

(84) Benannte Vertragsstaaten:
**DE ES FR GB IT**

(56) Entgegenhaltungen:
**EP-A- 0 020 907**
**EP-A- 0 353 580**
**GB-A- 464 657**
**GB-A- 1 185 111**
**US-A- 2 082 275**

(73) Patentinhaber: **HOECHST AKTIENGESELLSCHAFT**
**D-65926 Frankfurt am Main (DE)**

(72) Erfinder: **Aigner, Rudolf, DI (FH)**
**Hochkalterstrasse 7**
**W-8269 Burgkirchen (DE)**
Erfinder: **Maier, Guillermo, DI (FH)**
**Hauptstrasse 21**
**W-8161 Haiming (DE)**
Erfinder: **Müller, Rainer**
**Robert-Koch-Strasse 86**
**W-8363 Burghausen (DE)**
Erfinder: **Seitz, Hubert, Dr.**
**Talhauser Strasse 27**
**W-8369 Burgkirchen (DE)**

**EP 0 563 747 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von wäßrigen Lösungen von Betainen der allgemeinen Formel 1

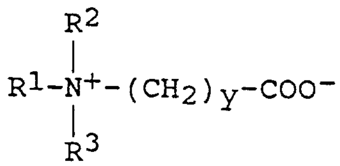

(1)

worin bedeuten: $R^1$ einen Alkylrest mit 6 bis 22 C-Atomen, vorzugsweise 8 bis 18 C-Atomen, oder einen Rest der Formel $R'CONH(CH_2)_z$-, worin R' ein Alkylrest mit 6 bis 22 C-atomen ist und z 2, 3 oder 4 ist, $R^2$ einen Alkylrest mit 1 bis 4 C-Atomen oder einen Rest der Formel $-(CH_2)_m-OH$, worin m 1, 2 oder 3 ist, $R^3$ einen Alkylrest mit 1 bis 4 C-Atomen oder einen Rest der genannten Formel $-(CH_2)_m-OH$ und y 1, 2 oder 3, durch Umsetzung von einem tertiären Amin der allgemeinen Formel 2

$R^1-NR^2R^3$ (2)

worin $R^1$, $R^2$ und $R^3$ die angegebenen Bedeutungen haben, mit einer ω-Halogencarbonsäure der allgemeinen Formel 3

$X-(CH_2)_y-COOH$ (3)

worin X ein Halogen ist, vorzugsweise Cl, und y die genannte Bedeutung hat,
und mit Alkalimetallhydroxid in wäßriger Phase.

Die Herstellung von wäßrigen Lösungen von Betainen durch Umsetzung (Quaternisierung) von tertiären Aminen mit einer ω-Halogencarbonsäure und Alkalimetallhydroxid in wäßriger Phase ist schon seit langem bekannt, zum Beispiel aus den beiden US-Patentschriften 3,819,539 und 4,497,825. Sie beruht auf der folgenden Gesamtreaktionsgleichung (die Reaktionskomponenten sind Dimethyllaurylamin, Monochloressigsäure und Natriumhydroxid):

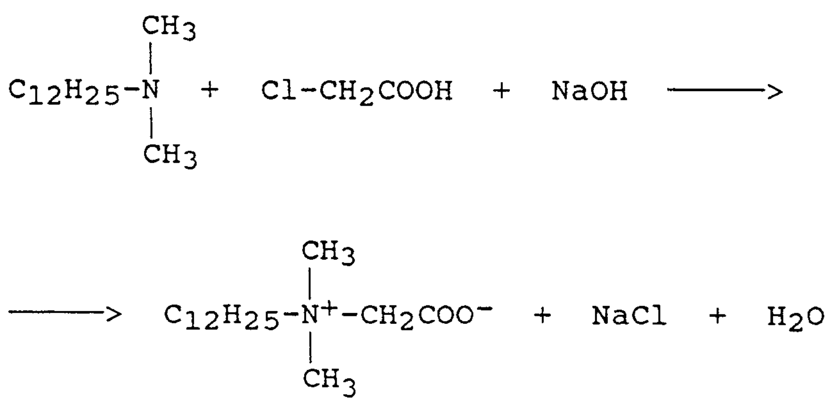

Die erhaltenen wäßrigen Lösungen bestehen im wesentlichen aus dem angestrebten Betain, dem gebildeten Alkalimetallhalogensalz und dem eingesetzten und gebildeten Wasser und haben im allgemeinen einen Wirkstoffgehalt von 20 bis 70 Gew.-%, vorzugsweise 25 bis 60 Gew.-%. Diese wäßrigen Betainlösungen stellen bereits als solche wertvolle Produkte dar, insbesondere für das Gebiet der Körperpflege. Betaine weisen nämlich nicht nur gute Reinigungseigenschaften, sondern auch gute Hautverträglichkeit auf. Die Hautverträglichkeit ist jedoch nur dann gegeben, wenn die Betainlösung nur noch sehr wenig Ausgangsamin enthält, da gerade davon negative Hauteinflüsse auszugehen scheinen.

Bei der Herstellung der in Rede stehenden wäßrigen Betainlösungen geht es also vor allem darum, einen möglichst hohen Umsatz des eingesetzten tertiären Amins mit gleichzeitig hoher Ausbeute an Betain

zu erzielen. Die erhaltene wäßrige Betainlösung sollte nämlich weniger als 2 Mol-%, vorzugsweise weniger als 1 Mol-%, (nicht umgesetztes) tertiäres Amin enthalten, bezogen auf eingesetztes tertiäres Amin (Ausgangsamin). Es ist schon häufig versucht worden, dieses Ziel durch spezielle Maßnahmen zu erreichen, so zum Beispiel durch Einhalten eines bestimmten pH-Wertes während der Quaternisierung, durch Zusammenbringen der Reaktionskomponenten tertiäres Amin, Halogencarbonsäure und Alkalimetallhydroxid in einer ganz bestimmten Reihenfolge, indem zum Beispiel das tertiäre Amin und die Halogencarbonsäure vorgelegt und das Alkalimetallhydroxid langsam zudosiert werden oder indem die Halogencarbonsäure und das Alkalimetallhydroxid vorgelegt und das tertiäre Amin zudosiert wird, ferner durch Verwendung spezieller Lösungsmittel und/oder durch Aufrechterhalten einer relativ niedrigen Temperatur während der Umsetzung. Mit den bekannten Verfahren wird also das angestrebte Ziel, die Aminverunreinigung der erhaltenen wäßrigen Betainlösungen auf die angegebenen Mol-%-Werte zu bringen, wenn überhaupt, nur durch mehr oder weniger umständliche Maßnahmen erreicht. Diese Verfahren sind ferner nur diskontinuierlich durchführbar.

Die Aufgabe der Erfindung besteht demnach darin, ein Verfahren zur Herstellung der eingangs genannten wäßrigen Betainlösungen zur Verfügung zu stellen, das die Betaine in hoher Ausbeute und hoher Reinheit liefert, das heißt wäßrige Betainlösungen mit weniger als 2 Mol-% Ausgangsamin, vorzugsweise weniger als 1 Mol-%, bezogen auf eingesetztes tertiäres Amin. Das neue Verfahren soll ferner in einfacher Weise kontinuierlich durchführbar sein.

Das erfindungsgemäße Verfahren ist dadurch gekennzeichnet, daß die Umsetzung in zwei oder drei kaskadenförmig angeordneten Rührkesseln kontinuierlich durchgeführt und dabei so vorgegangen wird, daß

a) dem ersten Rührkessel das tertiäre Amin und die ω-Halogencarbonsäure in einer Menge von 1 bis 1,3 mol pro mol tertiäres Amin, vorzugsweise 1 bis 1,15 mol pro mol tertiäres Amin, das Alkalimetallhydroxid in einer Menge von nur 0,80 bis 0,98 mol pro mol ω-Halogencarbonsäure, vorzugsweise 0,85 bis 0,95 mol pro mol ω-Halogencarbonsäure, und das Wasser in einer solchen Menge, daß der Betaingehalt der fertigen wäßrigen Betainlösung 20 bis 50 Gew.-% beträgt, vorzugsweise 25 bis 40 Gew.-%, gleichzeitig und kontinuierlich zugeführt werden und in diesem Kessel bei einer Temperatur von 60 bis 100 °C, vorzugsweise von 70 bis 95 °C, eine solche Verweilzeit eingehalten wird, daß das diesen Kessel verlassende und in den zweiten Rührkessel eintretende Produkt noch 2 bis 15 Mol-% tertiäres Amin enthält, vorzugsweise 3 bis 10 Mol-% tertiäres Amin, bezogen auf eingesetztes tertiäres Amin, und

b) dem zweiten Rührkessel eine weitere Menge an Alkalimetallhydroxid kontinuierlich zugeführt wird, die im Bereich von der beim ersten Kessel verbleibenden stöchiometrischen Alkalimetallhydroxid-Restmenge bis zu einem Überschuß von 5 Mol-% (bezogen auf eingesetzte ω-Halogencarbonsäure) liegt, vorzugsweise von 3 Mol-%, und in diesem Kessel bei einer Temperatur von 60 bis 100 °C, vorzugsweise 70 bis 95 °C, eine solche Verveilzeit eingehalten wird, daß die den Kessel verlassende wäßrige Betainlösung weniger als 2 Mol-% tertiäres Amin enthält, vorzugsweise weniger als 1 Mol-% tertiäres Amin, bezogen auf eingesetztes tertiäres Amin, wobei im Falle von drei Rührkesseln der dritte Kessel zu einer Nachreaktion bei einer Temperatur von 70 bis 100 °C, vorzugsweise 80 bis 100 °C, eingesetzt wird, um eine wäßrige Betainlösung zu erhalten, die einen im Vergleich zur Betainlösung vom zweiten Rührkessel noch niedrigeren Gehalt an tertiärem Ausgangsamin aufweist.

Das erfindungsgemäße Verfahren wird also kontinuierlich in zwei oder drei hintereinander angeordneten Rührkesseln durchgeführt, wobei die angestrebte (fertige) wäßrige Betainlösung dem zweiten Kessel oder dem dritten Kessel entnommen wird. Im ersten Rührkessel wird das Alkalimetallhydroxid nicht in der stöchiometrisch erforderlichen Menge - bezogen auf eingesetzte ω-Halogencarbonsäure - zudosiert, sondern in einem bestimmten Unterschuß, nämlich in einer Menge von 80 bis 98 Mol-%, vorzugsweise 85 bis 95 Mol-%. Im ersten Rührkessel wird ferner bis auf einen bestimmten Umsetzungsgrad gegangen. Die aus diesem Kessel fließende wäßrige Reaktionsmischung soll noch 2 bis 15 Mol-% vom eingesetzten tertiären Amin enthalten, vorzugsweise 3 bis 10 Mol-%. Im ersten Kessel wird also mit einem Unterschuß an Alkalimetallhydroxid umgesetzt und die Umsetzung bis zum angegebenen Amingehalt geführt. Im zweiten Rührkessel wird der Reaktionsmischung weiteres Alkalimetallhydroxid zudosiert, und zwar das beim ersten Rührkessel auf die stöchiometrische Menge verbleibende Alkalimetallhydroxid und gegebenenfalls darüber hinausgehendes Alkalimetallhydroxid bis zu einem stöchiometrischen Überschuß - bezogen auf eingesetzte ω-Halogencarbonsäure - von 5 Mol-%, vorzugsweise von 3 Mol-%. Das dem zweiten Reaktionskessel kontinuierlich zugeführte Alkalimetallhydroxid umfaßt also die genannte stöchiometrische Restmenge und den erwähnten Überschuß. Wenn man im zweiten Rührkessel eine wäßrige Betainlösung mit einem sehr niedrigen Gehalt an Ausgangsamin anstrebt, wird man im zweiten Rührkessel nicht nur die stöchiometrische Restmenge von Alkalimetallhydroxid zusetzen, sondern auch die genannte Überschußmenge; damit wird nämlich ein sehr hoher Umsatz des eingesetzten tertiären Amins bereits mit zwei Rührkesseln erreicht. Einen dritten Rührkessel wird man dann verwenden, wenn man eine besonders reine Betainlösung

herstellen möchte. Man wird dann die in den dritten Kessel eintretende wäßrige Betainlösung bei der angegebenen Temperatur, das sind 70 bis 100 °C, vorzugsweise 80 bis 100 °C, nachreagieren lassen bis der gewünschte niedrige Aminwert erreicht ist. Zur Erreichung dieser besonders niedrigen Aminwerte kann es vorteilhaft sein, auch noch im dritten Rührkessel Alkalimetallhydroxid kontinuierlich zuzusetzen. Der Zusatz von Alkalimetallhydroxid im dritten Rührkessel wird bevorzugt dann durchgeführt, wenn im zweiten Rührkessel nur die stöchiometrische Restmenge an Alkalimetallhydroxid zudosiert worden ist. In einem solchen Fall wird man also den erwähnten Überschuß an Alkalimetallhydroxid von bis zu 5 Mol-%, vorzugsweise von bis zu 3 Mol-%, bezogen auf eingesetzte Monohalogencarbonsäure, dem dritten Rührkessel zudosieren.

Die Umsetzung läuft aufgrund der einzuhaltenden Temperaturen bei Atmosphärendruck oder einem leichten Überdruck ab. Die mittlere Gesamtverweilzeit liegt bei etwa 10 bis 25 Stunden. Eine besondere Aufarbeitung der dem zweiten oder dritten Rührkessel entnommenen wäßrigen Betainlösung ist nicht erforderlich, da - wie eingangs erwähnt - diese Lösungen bereits als solche eingesetzt werden. Ihr pH-Wert wird in der Regel auf 7 bis 8 eingestellt, zum Beispiel durch Zugabe von Salzsäure.

Die tertiären Ausgangsamine entsprechen der eingangs angegebenen Formel 2. Der lange Alkylrest $R^1$ kann auch Doppelbindungen enthalten, vorzugsweise 1 bis 3. Bevorzugte Ausgangsamine sind solche der Formel 2, wenn $R^1$ ein Alkylrest mit 8 bis 18 C-Atomen ist oder ein Rest der Formel $R'CONH(CH_2)_z$-, worin R' ein Alkylrest mit 8 bis 18 C-Atomen ist und z 2, 3 oder 4 ist, und $R^2$ und $R^3$ jeweils Methyl sind. Als Beispiele seien genannt: Dimethyloctylamin, Dimethyllaurylamin, Dimethylstearylamin, Dimethylcocosalkylamin, Dimethyltalgalkylamin und dergleichen sowie Lauroylaminopropyldimethylamin, Stearoylaminopropyldimethylamin, Cocosacylaminopropyldimethylamin und dergleichen. Die $\omega$-Halogencarbonsäure ist vorzugsweise die Monochloressigsäure. Das Alkalimetallhydroxid ist vorzugsweise Natriumhydroxid oder Kaliumhydroxid. Das Alkalimetallhydroxid wird vorzugsweise in Form einer wäßrigen Lösung eingesetzt, und zwar in einer 15- bis 70gew.%igen wäßrigen Lösung, vorzugsweise in einer 20- bis 50gew.%igen Lösung. Die gesamte Menge an Wasser (Lösungsmittel) wird, wie oben bereits beschrieben, so gewählt, daß die Konzentration an Wirkstoff (Betain) in der fertigen wäßrigen Betainlösung etwa 20 bis 50 Gew.-%, vorzugsweise 25 bis 40 Gew.-%, beträgt. Der Ausdruck "wäßrige" Betainlösung umfaßt auch solche Lösungen, die neben Wasser auch andere Lösungsmittel enthalten, zum Beispiel Methanol, Ethanol, Propanol und/oder Isopropanol.

Das erfindungsgemäße Verfahren weist eine Reihe von Vorteilen auf. Es ist kontinuierlich durchführbar und ergibt im Vergleich zu diskontinuierlichen Verfahren eine wesentlich höhere Raum-Zeit-Ausbeute. Es liefert ferner einen praktisch vollständigen Umsatz des eingesetzten tertiären Amins und gleichzeitig sehr reine wäßrige Betainlösungen. So können wäßrige Betainlösungen erhalten werden, die weniger als 2 Mol-% oder weniger als 1 Mol-% tertiäres Amin enthalten, bezogen auf die eingesetzte Molmenge an tertiärem Amin, oder auch solche Betainlösungen, die praktisch frei sind von tertiärem Ausgangsamin. Die Betainlösungen sind auch sehr rein bezüglich Halogencarbonsäure und deren Salzen, zum Beispiel Monochloressigsäuresalz, sowie bezüglich Hydroxycarbonsäuren, zum Beispiel Glykolsäure. Mit dem erfindungsgemäßen Verfahren ist es also in einfacher Weise möglich, das Betain in hoher Ausbeute und hoher Reinheit herzustellen. Die Erfindung wird nun an Beispielen noch näher erläutert.

Beispiel 1

Die Umsetzung wurde in zwei kaskadenförmig angeordneten Rührkesseln mit je 8 $m^3$ Volumen durchgeführt. Jeder der beiden Kessel war mit einem Rührer, einem Thermometer und einem Kühlmantel (Abführung der freiwerdenden Reaktionswärme mittels Kühlwasser) versehen. Dem ersten Rührkessel wurden über vier Einlaufstutzen pro Stunde kontinuierlich zugeführt: 226 kg (1,0 kmol) Dimethylalkylamin (Alkyl = circa 70 % $C_{12}$, 25 % $C_{14}$ und 5 % $C_{16}$), 118,1 kg (1,0 kmol) 80%ige MCE (MCE = Monochloressigsäure) und 68 kg 50gew.%ige wäßrige Natronlauge, das sind 0,85 kmol NaOH pro kmol MCE, und noch weiteres Wasser, nämlich 712 kg, so daß eine circa 25gew.%ige Lösung an Wirkstoff (Betain) entstand. Nachdem die stöchiometrische Menge an NaOH, bezogen auf eingesetzte MCE, 1,0 kmol beträgt, wurden mit den 0,85 kmol NaOH pro kmol MCE dem ersten Rührkessel nur 85 Mol-% von der stöchiometrisch erforderlichen Menge zudosiert, das entspricht einem stöchiometrischen Alkaliunterschied von 0,15 kmol oder 15 Mol-%. Die beim ersten Rührkessel fehlende Alkalirestmenge von 0,15 kmol (das sind 12,0 kg 50gew.%ige Natronlauge) wurde pro Stunde kontinuierlich dem zweiten Rührkessel zugeführt. Im ersten Rührkessel wurde eine Temperatur von 95 °C und im zweiten Kessel eine Temperatur von ebenfalls 95 °C gehalten (beide Kessel wurden unter Atmosphärendruck betrieben). Die mittlere Verweilzeit über beide Rührkessel betrug 14 Stunden. Das den ersten Kessel verlassende Produkt (Produktgemisch) hatte einen Gehalt an freiem Amin von 9 Mol-% und das den zweiten Kessel verlassende Produkt einen

Gehalt von 1,8 Mol-%, Molprozente bezogen auf das eingesetzte Dimethylalkylamin.

Beispiel 2

Die Umsetzung wurde in den beiden in Beispiel 1 angegebenen Rührkesseln durchgeführt. Dem ersten Rührkessel wurden pro Stunde kontinuierlich zugeführt: 220 kg (1,0 kmol) Dimethyllaurylamin (Lauryl = > 98 % $C_{12}$), 129,9 kg (1,1 kmol) 80%ige MCE und 79,2 kg 50gew.%ige wäßrige Natronlauge, das sind 0,9 kmol NaOH pro kmol MCE, und noch weiteres Wasser, nämlich 425 kg, so daß eine circa 35gew.%ige Lösung an Betain entstand. Nachdem die stöchiometrische Menge an NaOH, bezogen auf eingesetzte MCE, 1,1 kmol beträgt, wurden mit den 0,9 kmol NaOH pro kmol MCE dem ersten Rührkessel nur 90 Mol-% von der stöchiometrisch erforderlichen Menge zudosiert, das entspricht einem stöchiometrischen Alkaliunterschied von 0,10 kmol oder 10 Mol-%. Die beim ersten Rührkessel fehlende Alkalirestmenge von 0,10 kmol (das sind 8,8 kg 50gew.%ige Natronlauge) wurde pro Stunde kontinuierlich dem zweiten Rührkessel zugeführt. Im ersten Rührkessel wurde eine Temperatur von 70 °C und im zweiten Kessel eine Temperatur von ebenfalls 70 °C gehalten (beide Kessel wurden unter Atmosphärendruck betrieben). Die mittlere Verweilzeit über beide Rührkessel betrug 18 Stunden. Das den ersten Kessel verlassende Produkt hatte einen Gehalt an freiem Amin von 5 Mol-% und das den zweiten Kessel verlassende Produkt einen Gehalt von 0,9 Mol-%, Molprozente bezogen auf das eingesetzte Dimethyllaurylamin.

Beispiel 3

Die Umsetzung wurde in den beiden in Beispiel 1 angegebenen Rührkesseln durchgeführt. Dem ersten Rührkessel wurden pro Stunde kontinuierlich zugeführt: 226 kg (1,0 kmol) Dimethylalkylamin (Alkyl = circa 70 % $C_{12}$, 25 % $C_{14}$ und 5 % $C_{16}$), 135,8 kg (1,15 kmol) 80%ige MCE und 87,5 kg 50gew.%ige wäßrige Natronlauge, das sind 0,95 kmol NaOH pro kmol MCE, und noch weiteres Wasser, nämlich 462 kg, so daß eine circa 30gew.%ige Lösung an Betain entstand. Nachdem die stöchiometrische Menge an NaOH, bezogen auf eingesetzte MCE, 1,15 kmol beträgt, wurden mit den 0,95 kmol NaOH pro kmol MCE dem ersten Rührkessel nur 95 Mol-% von der stöchiometrisch erforderlichen Menge zudosiert, das entspricht einem stöchiometrischen Alkaliunterschied von 0,05 kmol oder 5 Mol-%. Die beim ersten Rührkessel fehlende Alkalirestmenge von 0,05 kmol und zusätzliche 0,03 kmol NaOH als Überschuß (das sind 7,4 kg 50gew.%ige Natronlauge) wurden pro Stunde kontinuierlich dem zweiten Rührkessel zugeführt. Im ersten Rührkessel wurde eine Temperatur von 95 °C und im zweiten Kessel eine Temperatur von ebenfalls 95 °C gehalten (beide Kessel wurden unter Atmosphärendruck betrieben). Die mittlere Verweilzeit über beide Rührkessel betrug 17 Stunden. Das den ersten Kessel verlassende Produkt hatte einen Gehalt an freiem Amin von 3 Mol-% und das den zweiten Kessel verlassende Produkt einen Gehalt von 0,5 Mol-%, Molprozente bezogen auf das eingesetzte Dimethylalkylamin.

Beispiel 4

Die Umsetzung wurde in drei kaskadenförmig angeordneten Rührkesseln mit je 8 $m^3$ Volumen durchgeführt. Jeder der drei Kessel war mit einem Rührer, einem Thermometer und einem Kühlmantel versehen. Dem ersten Rührkessel wurden über vier Einlaufstutzen pro Stunde kontinuierlich zugeführt: 226 kg (1,0 kmol) Dimethylalkylamin (Alkyl = circa 70 % $C_{12}$, 25 % $C_{14}$ und 5 % $C_{16}$), 124,1 kg (1,05 kmol) 80%ige MCE und 79,8 kg 50gew.%ige wäßrige Natronlauge, das sind 0,95 kmol NaOH pro kmol MCE, und noch weiteres Wasser, nämlich 462 kg, so daß eine circa 30gew.%ige Lösung an Betain entstand. Nachdem die stöchiometrische Menge an NaOH, bezogen auf eingesetzte MCE, 1,05 kmol beträgt, wurden mit den 0,95 kmol NaOH pro kmol MCE dem ersten Rührkessel nur 95 Mol-% von der stöchiometrisch erforderlichen Menge zudosiert, das entspricht einem stöchiometrischen Alkaliunterschied von 0,05 kmol oder 5 Mol-%. Die beim ersten Rührkessel fehlende Alkalirestmenge von 0,05 kmol (das sind 4,2 kg 50gew.%ige Natronlauge) wurde pro Stunde kontinuierlich dem zweiten Rührkessel zugeführt. Dem dritten Rührkessel wurde noch ein Überschuß von 0,03 kmol Alkali, bezogen auf MCE, zugeführt (das sind 2,5 kg 50gew.%ige Natronlauge/Stunde). Im ersten Rührkessel wurde eine Temperatur von 90 °C, im zweiten Kessel eine Temperatur von 95 °C und im dritten Kessel eine Temperatur von 98 °C gehalten (alle Kessel wurden unter Atmosphärendruck betrieben). Die mittlere Verweilzeit über alle Rührkessel betrug 25 Stunden. Das den ersten Kessel verlassende Produkt hatte einen Gehalt an freiem Amin von 6 Mol-%, das den zweiten Kessel verlassende Produkt einen Gehalt von 0,6 Mol-% und das den dritten Kessel verlassende Produkt einen Gehalt an freiem Amin von 0,2 Mol-%, Molprozente bezogen auf das eingesetzte Dimethylalkylamin.

Beispiel 5

Die Umsetzung wurde in den drei in Beispiel 4 angegebenen Rührkesseln durchgeführt. Dem ersten Rührkessel wurden über vier Einlaufstutzen pro Stunde kontinuierlich zugeführt: 317 kg (1,0 kmol) N-Cocosfettsäureamidopropyl-N,N-dimethylamin (Amidamin), 129,9 kg (1,1 kmol) 80%ige MCE und 83,6 kg 50gew.%ige wäßrige Natronlauge, das sind 0,95 kmol NaOH pro kmol MCE, und noch weiteres Wasser, nämlich 745 kg, so daß eine circa 30gew.%ige Lösung an Betain entstand. Nachdem die stöchiometrische Menge an NaOH, bezogen auf eingesetzte MCE, 1,10 kmol beträgt, wurden mit den 0,95 kmol NaOH pro kmol MCE dem ersten Rührkessel nur 95 Mol-% von der stöchiometrisch erforderlichen Menge zudosiert, das entspricht einem stöchiometrischen Alkaliunterschied von 0,05 kmol oder 5 Mol-%. Die beim ersten Rührkessel fehlende Alkalirestmenge von 0,05 kmol (das sind 4,4 kg 50gew.%ige Natronlauge) wurde pro Stunde kontinuierlich dem zweiten Rührkessel zugeführt. Dem dritten Rührkessel wurde noch ein Überschuß von 0,05 kmol Alkali, bezogen auf MCE, zugeführt (das sind 4,4 kg 50gew.%ige Natronlauge/Stunde). Im ersten Rührkessel wurde eine Temperatur von 90 °C, im zweiten Kessel eine Temperatur von 95 °C und im dritten Kessel eine Temperatur von 98 °C gehalten (alle Kessel wurden unter Atmosphärendruck betrieben). Die mittlere Verweilzeit über alle Rührkessel betrug 18 Stunden. Das den ersten Kessel verlassende Produkt hatte einen Gehalt an freiem Amin von 4 Mol-%, das den zweiten Kessel verlassende Produkt einen Gehalt von 0,4 Mol-% und das den dritten Kessel verlassende Produkt einen Gehalt an freiem Amin von 0,2 Mol-%, Molprozente bezogen auf das eingesetzte Amidamin.

Die Beispiele 1 bis 5 sind in der nachstehenden Tabelle zusammengefaßt.

TABELLE

| Bei-spiel | tertiäres Ausgangsamin (kmol) | MCE (kmol) | NaOH bezogen auf MCE (kmol) | | | Restamin, bezogen auf eingesetztes Amin (Mol-%) | | | Temperatur (°C) | Verweilzeit (Stunden) | Betaingehalt (Gew.-%) |
|---|---|---|---|---|---|---|---|---|---|---|---|
| | | | 1. K | 2. K | 3. K | 1. K | 2. K | 3. K | | | |
| 1 | 1 | 1 | 0,85 | 0,15 | --- | 9 | 1,8 | --- | 95/95 | 14 | 25 |
| 2 | 1 | 1,10 | 0,90 | 0,10 | --- | 5 | 0,9 | --- | 70/70 | 18 | 35 |
| 3 | 1 | 1,15 | 0,95 | 0,08 | --- | 3 | 0,5 | --- | 95/95 | 17 | 30 |
| 4 | 1 | 1,05 | 0,95 | 0,05 | 0,03 | 6 | 0,6 | 0,2 | 90/95/98 | 25 | 30 |
| 5 | 1 | 1,10 | 0,95 | 0,05 | 0,05 | 4 | 0,4 | 0,2 | 90/95/98 | 18 | 30 |

MCE = Monochloressigsäure

K = Rührkessel

## Patentansprüche

**1.** Verfahren zur Herstellung von wäßrigen Lösungen von Betainen der allgemeinen Formel 1

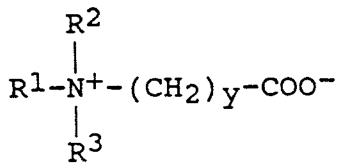

(1)

worin bedeuten: $R^1$ einen Alkylrest mit 6 bis 22 C-Atomen oder einen Rest der Formel R'CONH-$(CH_2)_z$-, worin R' ein Alkylrest mit 6 bis 22 C-atomen ist und z 2, 3 oder 4 ist, $R^2$ einen Alkylrest mit 1 bis 4 C-Atomen oder einen Rest der Formel $-(CH_2)_m-OH$, worin m 1, 2 oder 3 ist, $R^3$ einen Alkylrest mit 1 bis 4 C-Atomen oder einen Rest der genannten Formel $-(CH_2)_m-OH$ und y 1, 2 oder 3,
durch Umsetzung von einem tertiären Amin der allgemeinen Formel 2

$R^1-NR^2R^3$ (2)

worin $R^1$, $R^2$ und $R^3$ die angegebenen Bedeutungen haben,
mit einer ω-Halogencarbonsäure der allgemeinen Formel 3

$X-(CH_2)_y-COOH$ (3)

worin X ein Halogen ist und y die genannte Bedeutung hat,
und mit Alkalimetallhydroxid in wäßriger Phase, dadurch gekennzeichnet, daß die Umsetzung in zwei oder drei kaskadenförmig angeordneten Rührkesseln kontinuierlich durchgeführt und dabei so vorgegangen wird, daß
a) dem ersten Rührkessel das tertiäre Amin und die ω-Halogencarbonsäure in einer Menge von 1 bis 1,3 mol pro mol tertiäres Amin, das Alkalimetallhydroxid in einer Menge von nur 0,80 bis 0,98 mol pro mol ω-Halogencarbonsäure und das Wasser in einer solchen Menge, daß der Betaingehalt der fertigen wäßrigen Betainlösung 20 bis 50 Gew.-% beträgt, gleichzeitig und kontinuierlich zugeführt werden und in diesem Kessel bei einer Temperatur von 60 bis 100 °C eine solche Verweilzeit eingehalten wird, daß das diesen Kessel verlassende und in den zweiten Rührkessel eintretende Produkt noch 2 bis 15 Mol-% tertiäres Amin enthält, bezogen auf eingesetztes tertiäres Amin, und
b) dem zweiten Rührkessel eine weitere Menge an Alkalimetallhydroxid kontinuierlich zugeführt wird, die im Bereich von der beim ersten Kessel verbleibenden stöchiometrischen Alkalimetallhydroxid-Restmenge bis zu einem Überschuß von 5 Mol-% liegt, und in diesem Kessel bei einer Temperatur von 60 bis 100 °C eine solche Verweilzeit eingehalten wird, daß die den Kessel verlassende wäßrige Betainlösung weniger als 2 Mol-% tertiäres Amin enthält, bezogen auf eingesetztes tertiäres Amin, wobei im Falle von drei Rührkesseln der dritte Kessel zu einer Nachreaktion bei einer Temperatur von 70 bis 100 °C eingesetzt wird, um eine wäßrige Betainlösung zu erhalten, die einen im Vergleich zur Betainlösung vom zweiten Rührkessel noch niedrigeren Gehalt an tertiärem Ausgangsamin aufweist.

**2.** Verfahren nach Anspruch 1, dadurch gekennzeichnet, daß
a) dem ersten Rührkessel das tertiäre Amin und die ω-Halogencarbonsäure in einer Menge von 1 bis 1,15 mol pro mol tertiäres Amin, das Alkalimetallhydroxid in einer Menge von nur 0,85 bis 0,95 mol pro mol ω-Halogencarbonsäure und das Wasser in einer solchen Menge, daß der Betaingehalt der fertigen wäßrigen Betainlösung 25 bis 40 Gew.-% beträgt, gleichzeitig und kontinuierlich zugeführt werden und in diesem Kessel bei einer Temperatur von 70 bis 95 °C eine solche Verweilzeit eingehalten wird, daß das diesen Kessel verlassende und in den zweiten Rührkessel eintretende Produkt noch 3 bis 10 Mol-% tertiäres Amin enthält, bezogen auf eingesetztes tertiäres Amin, und
b) dem zweiten Rührkessel eine weitere Menge an Alkalimetallhydroxid kontinuierlich zugeführt wird, die im Bereich von der beim ersten Kessel verbleibenden stöchiometrischen Alkalimetallhydroxid-Restmenge bis zu einem Überschuß von 3 Mol-% liegt, und in diesem Kessel bei einer Temperatur von 70 bis 95 °C eine solche Verweilzeit eingehalten wird, daß die den Kessel verlassende wäßrige

Betainlösung weniger als 2 Mol-% tertiäres Amin enthält, bezogen auf eingesetztes tertiäres Amin, wobei im Falle von drei Rührkesseln der dritte Kessel zu einer Nachreaktion bei einer Temperatur von 80 bis 100 °C eingesetzt wird, um eine wäßrige Betainlösung zu erhalten, die einen im Vergleich zur Betainlösung vom zweiten Rührkessel noch niedrigeren Gehalt an tertiärem Ausgangsamin aufweist.

**3.** Verfahren nach Anspruch 1, dadurch gekennzeichnet, daß die Umsetzung in zwei kaskadenförmig angeordneten Rührkesseln kontinuierlich durchgeführt und dabei so vorgegangen wird, daß

a) dem ersten Rührkessel das tertiäre Amin und die ω-Halogencarbonsäure in einer Menge von 1 bis 1,15 mol pro mol tertiäres Amin, das Alkalimetallhydroxid in einer Menge von nur 0,85 bis 0,95 mol pro mol ω-Halogencarbonsäure und das Wasser in einer solchen Menge, daß der Betaingehalt der fertigen wäßrigen Betainlösung 25 bis 40 Gew.-% beträgt, gleichzeitig und kontinuierlich zugeführt werden und in diesem Kessel bei einer Temperatur von 70 bis 95 °C eine solche Verweilzeit eingehalten wird, daß das diesen Kessel verlassende und in den zweiten Rührkessel eintretende Produkt noch 3 bis 10 Mol-% tertiäres Amin enthält, bezogen auf eingesetztes tertiäres Amin, und

b) dem zweiten Rührkessel eine weitere Menge an Alkalimetallhydroxid kontinuierlich zugeführt wird, die im Bereich von der beim ersten Kessel verbleibenden stöchiometrischen Alkalimetallhydroxid-Restmenge bis zu einem Überschuß von 3 Mol-% liegt, und in diesem Kessel bei einer Temperatur von 70 bis 95 °C eine solche Verweilzeit eingehalten wird, daß die den Kessel verlassende wäßrige Betainlösung weniger als 2 Mol-% tertiäres Amin enthält, bezogen auf eingesetztes tertiäres Amin.

**4.** Verfahren nach Anspruch 1, dadurch gekennzeichnet, daß die Umsetzung in drei kaskadenförmig angeordneten Rührkesseln kontinuierlich durchgeführt und dabei so vorgegangen wird, daß

a) dem ersten Rührkessel das tertiäre Amin und die ω-Halogencarbonsäure in einer Menge von 1 bis 1,15 mol pro mol tertiäres Amin, das Alkalimetallhydroxid in einer Menge von nur 0,85 bis 0,95 mol pro mol ω-Halogencarbonsäure und das Wasser in einer solchen Menge, daß der Betaingehalt der fertigen wäßrigen Betainlösung 25 bis 40 Gew.-% beträgt, gleichzeitig und kontinuierlich zugeführt werden und in diesem Kessel bei einer Temperatur von 70 bis 95 °C eine solche Verweilzeit eingehalten wird, daß das diesen Kessel verlassende und in den zweiten Rührkessel eintretende Produkt noch 3 bis 10 Mol-% tertiäres Amin enthält, bezogen auf eingesetztes tertiäres Amin,

b) dem zweiten Rührkessel die beim ersten Kessel verbleibende stöchiometrische Alkalimetallhydroxid-Restmenge kontinuierlich zugeführt wird und in diesem Kessel bei einer Temperatur von 70 bis 95 °C eine solche Verweilzeit eingehalten wird, daß das diesen Kessel verlassende und in den dritten Kessel eintretende Produkt weniger als 2 Mol-% tertiäres Amin enthält, bezogen auf eingesetztes tertiäres Amin, und

c) dem dritten Rührkessel eine weitere Menge an Alkalimetallhydroxid bis zu einem stöchiometrischen Überschuß von 3 Mol-% kontinuierlich zugeführt wird und in diesem Kessel bei einer Temperatur von 80 bis 100 °C eine solche Verweilzeit eingehalten wird, daß die den Kessel verlassende wäßrige Betainlösung noch weniger Molprozent tertiäres Amin enthält als die in den Kessel eintretende Betainlösung.

**5.** Verfahren nach einem der Ansprüche 1 bis 4, dadurch gekennzeichnet, daß im zweiten Rührkessel eine solche Verweilzeit eingehalten wird, daß die den Kessel verlassende wäßrige Betainlösung weniger als 1 Mol-% tertiäres Amin enthält, bezogen auf eingesetztes tertiäres Amin.

**6.** Verfahren nach einem der Ansprüche 1 bis 5, dadurch gekennzeichnet, daß als tertiäres Amin ein solches der Formel 2 eingesetzt wird, worin bedeuten: $R^1$ einen Alkylrest mit 8 bis 18 C-Atomen oder einen Rest der Formel $R'CONH(CH_2)_z$-, worin R' ein Alkylrest mit 8 bis 18 C-atomen ist und z 2, 3 oder 4 ist, $R^2$ einen Alkylrest mit 1 bis 4 C-Atomen und $R^3$ ebenfalls einen Alkylrest mit 1 bis 4 C-Atomen, als ω-Halogencarbonsäure die Monochloressigsäure und als Alkalimetallhydroxid Kaliumhydroxid oder Natriumhydroxid eingesetzt wird.

## Claims

**1.** A process for the preparation of aqueous solutions of betaines of the formula 1

$$\begin{array}{c} R^2 \\ | \\ R^1\text{-}N^{+}\text{-}(CH_2)_y\text{-}COO^{-} \\ | \\ R^3 \end{array} \qquad (1)$$

in which: $R^1$ is an alkyl radical having 6 to 22 carbon atoms or is a radical of the formula R'CONH-$(CH_2)_z$-, in which R' is an alkyl radical having 6 to 22 carbon atoms and z is 2, 3 or 4, $R^2$ is an alkyl radical having 1 to 4 carbon atoms or is a radical of the formula $-(CH_2)_m-OH$, in which m is 1, 2 or 3, $R^3$ is an alkyl radical having 1 to 4 carbon atoms or is a radical of said formula $-(CH_2)_m-OH$ and y is 1, 2 or 3,
by reaction of a tertiary amine of the formula 2

$R^1-NR^2R^3$ (2)

in which $R^1$, $R^2$ and $R^3$ have the meanings given above,
with an ω-halocarboxylic acid of the formula 3

$X-(CH_2)_y-COOH$ (3)

in which X is a halogen and y has the above-mentioned meaning,
and with alkali metal hydroxide in the aqueous phase, which comprises carrying out the reaction continuously in two or three stirred tanks arranged in a cascade and proceeding in such a way that
a) the first stirred tank is supplied simultaneously and continuously with the tertiary amine and the ω-halocarboxylic acid in an amount of 1 to 1.3 mol per mole of tertiary amine, the alkali metal hydroxide in an amount of only 0.80 to 0.98 mol per mole of ω-halocarboxylic acid and the water in an amount such that the betaine content of the finished aqueous betaine solution is 20 to 50% by weight, and in this tank at a temperature of 60 to 100 °C a residence time is maintained such that the product leaving this tank and entering the second stirred tank still contains 2 to 15 mol-% of tertiary amine, based on tertiary amine used, and
b) the second stirred tank is continuously supplied with a further amount of alkali metal hydroxide which is in the range from the remainder of the stoichiometric amount of alkali metal hydroxide remaining from the first tank up to an excess of 5 mol-%, and in this tank at a temperature of 60 to 100 °C a residence time is maintained such that the aqueous betaine solution leaving the tank contains less than 2 mol-% of tertiary amine, based on tertiary amine used, where, when three stirred tanks are used, the third tank is used for a following reaction at a temperature of 70 to 100 °C in order to obtain an aqueous betaine solution which, in comparison with the betaine solution from the second stirred tank, has a still lower content of tertiary starting amine.

**2.** The process as claimed in claim 1, wherein
a) the first stirred tank is supplied simultaneously and continuously with the tertiary amine and the ω-halocarboxylic acid in an amount of 1 to 1.15 mol per mole of tertiary amine, the alkali metal hydroxide in an amount of only 0.85 to 0.95 mol per mole of ω-halocarboxylic acid and the water in an amount such that the betaine content of the finished aqueous betaine solution is 25 to 40% by weight, and in this tank at a temperature of 70 to 95 °C a residence time is maintained such that the product leaving this tank and entering the second stirred tank still contains 3 to 10 mol-% of tertiary amine, based on tertiary amine used, and
b) the second stirred tank is continuously supplied with a further amount of alkali metal hydroxide which is in the range from the remainder of the stoichiometric amount of alkali metal hydroxide remaining from the first tank up to an excess of 3 mol-%, and in this tank at a temperature of 70 to 95 °C a residence time is maintained such that the aqueous betaine solution leaving the tank contains less than 2 mol-% of tertiary amine, based on tertiary amine used, where, when three stirred tanks are used, the third tank is used for a following reaction at a temperature of 80 to 100 °C

in order to obtain an aqueous betaine solution which, in comparison with the betaine solution from the second stirred tank, has a still lower content of tertiary starting amine.

**3.** The process as claimed in claim 1, which comprises carrying out the reaction continuously in two stirred tanks arranged in a cascade and proceeding in such a way that

a) the first stirred tank is supplied simultaneously and continuously with the tertiary amine and the ω-halocarboxylic acid in an amount of 1 to 1.15 mol per mole of tertiary amine, the alkali metal hydroxide in an amount of only 0.85 to 0.95 mol per mole of ω-halocarboxylic acid and the water in an amount such that the betaine content of the finished aqueous betaine solution is 25 to 40% by weight, and in this tank at a temperature of 70 to 95 °C a residence time is maintained such that the product leaving this tank and entering the second stirred tank still contains 3 to 10 mol-% of tertiary amine, based on tertiary amine used, and

b) the second stirred tank is continuously supplied with a further amount of alkali metal hydroxide which is in the range from the residue of the stoichiometric amount of alkali metal hydroxide remaining from the first tank up to an excess of 3 mol-%, and in this tank at a temperature of 70 to 95 °C a residence time is maintained such that the aqueous betaine solution leaving the tank contains less than 2 mol-% of tertiary amine, based on tertiary amine used.

**4.** The process as claimed in claim 1, which comprises carrying out the reaction continuously in three stirred tanks arranged in a cascade and proceeding in such a way that

a) the first stirred tank is supplied simultaneously and continuously with the tertiary amine and the ω-halocarboxylic acid in an amount of 1 to 1.15 mol per mole of tertiary amine, the alkali metal hydroxide in an amount of only 0.85 to 0.95 mol per mole of ω-halocarboxylic acid and the water in an amount such that the betaine content of the finished aqueous betaine solution is 25 to 40% by weight, and in this tank at a temperature of 70 to 95 °C a residence time is maintained such that the product leaving this tank and entering the second stirred tank still contains 3 to 10 mol-% of tertiary amine, based on tertiary amine used,

b) the second stirred tank is continuously supplied with the remainder of the stoichiometric amount of alkali metal hydroxide remaining from the first tank, and in this tank at a temperature of 70 to 95 °C a residence time is maintained such that the product leaving this tank and entering the third tank contains less than 2 mol-% of tertiary amine, based on tertiary amine used, and

c) the third stirred tank is continuously supplied with a further amount of alkali metal hydroxide up to a stoichiometric excess of 3 mol-%, and in this tank at a temperature of 80 to 100 °C a residence time is maintained such that the aqueous betaine solution leaving the tank contains a still lower molar percentage of tertiary amine than the betaine solution entering the tank.

**5.** The process as claimed in one of claims 1 to 4, wherein a residence time is maintained in the second stirred tank such that the aqueous betaine solution leaving the tank contains less than 1 mol-% of tertiary amine, based on tertiary amine used.

**6.** The process as claimed in one of claims 1 to 5, wherein the tertiary amine used is one of those of the formula 2, in which: $R^1$ is an alkyl radical having 8 to 18 carbon atoms or is a radical of the formula $R'CONH(CH_2)_z$-, in which R' is an alkyl radical having 8 to 18 carbon atoms and z is 2, 3 or 4, $R^2$ is an alkyl radical having 1 to 4 carbon atoms and $R^3$ is likewise an alkyl radical having 1 to 4 carbon atoms, the ω-halocarboxylic acid used is monochloroacetic acid and the alkali metal hydroxide used is potassium hydroxide or sodium hydroxide.

**Revendications**

**1.** Procédé de préparation de solutions aqueuses de bétaïnes de formule générale 1

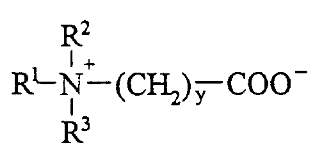

(1)

dans laquelle

$R^1$ représente un reste alkyle de 6 à 22 atomes de carbone ou un reste de formule $R'CONH(CH_2)_z$- dans laquelle R' représente un reste alkyle de 6 à 22 atomes de carbone et z est 2, 3 ou 4,

$R^2$ représente un reste alkyle de 1 à 4 atomes de carbone ou un reste de formule $-(CH_2)_m$-OH dans laquelle m est 1, 2 ou 3,

$R^3$ représente un reste alkyle de 1 à 4 atomes de carbone ou un reste de la formule précitée $-(CH_2)_m$-OH, et

y est 1, 2 ou 3,

par réaction d'une amine tertiaire de formule générale 2

$R^1$-$NR^2R^3$ (2)

dans laquelle $R^1$, $R^2$, et $R^3$ ont les significations indiquées, avec un acide ω-halogénocarboxylique de formule générale 3

X-$(CH_2)_y$-COOH (3)

dans laquelle X est un halogène et y a la signification indiquée, et avec un hydroxyde de métal alcalin en phase aqueuse, caractérisé en ce que l'on effectue la réaction en continu dans deux ou trois réacteurs à agitation disposés en cascade, et en ce que l'on procède de façon à

a) introduire en même temps et en continu dans le premier réacteur à agitation l'amine tertiaire et l'acide ω-halogénocarboxylique en une quantité de 1 à 1,3 mole par mole d'amine tertiaire, l'hydroxyde de métal alcalin en une quantité de seulement 0,80 à 0,98 mole par mole d'acide ω-halogénocarboxylique, et l'eau en une quantité telle que la teneur en bétaïne de la solution aqueuse de bétaïne finale soit de 20 à 50 % en masse, et maintenir dans ce réacteur, à une température de 60 à 100 °C, un temps de séjour tel que le produit quittant ce réacteur et entrant dans le deuxième réacteur à agitation contienne encore 2 à 15 % en moles d'amine tertiaire, par rapport à l'amine tertiaire introduite, et à

b) introduire en continu dans le deuxième réacteur à agitation une nouvelle quantité d'hydroxyde de métal alcalin, qui correspond à la différence entre une quantité allant de la quantité stoechiométrique d'hydroxyde de métal alcalin à un excès de 5 % en moles et celle introduite dans le premier réacteur, et maintenir dans ce réacteur, à une température de 60 à 100 °C, un temps de séjour tel que la solution aqueuse de bétaïne quittant le réacteur contienne moins de 2 % en moles d'amine tertiaire, par rapport à l'amine tertiaire introduite, et, dans le cas de trois réacteurs à agitation, utiliser le troisième réacteur pour une réaction ultérieure à une température de 70 à 100 °C pour obtenir une solution aqueuse de bétaïne qui présente une teneur en amine tertiaire de départ encore inférieure par comparaison à la solution de bétaïne du deuxième réacteur à agitation.

**2.** Procédé selon la revendication 1, caractérisé en ce que

a) on introduit en même temps et en continu dans le premier réacteur à agitation l'amine tertiaire et l'acide ω-halogénocarboxylique en une quantité de 1 à 1,15 mole par mole d'amine tertiaire, l'hydroxyde de métal alcalin en une quantité de seulement 0,85 à 0,95 mole par mole d'acide ω-halogénocarboxylique, et l'eau en une quantité telle que la teneur en bétaïne de la solution aqueuse de bétaïne finale soit de 25 à 40 % en masse, et on maintient dans ce réacteur, à une température de 70 à 95 °C, un temps de séjour tel que le produit quittant ce réacteur et entrant dans le deuxième réacteur à agitation contienne encore 3 à 10 % en moles d'amine tertiaire, par rapport à l'amine tertiaire introduite, et

b) on introduit en continu dans le deuxième réacteur à agitation une nouvelle quantité d'hydroxyde de métal alcalin, qui correspond à la différence entre une quantité allant de la quantité stoechiométrique d'hydroxyde de métal alcalin à un excès de 3 % en moles et celle introduite dans le premier réacteur, et on maintient dans ce réacteur, à une température de 70 à 95 °C, un temps de séjour tel que la solution aqueuse de bétaïne quittant le réacteur contienne moins de 2 % en moles d'amine tertiaire, par rapport à l'amine tertiaire introduite, et, dans le cas de trois réacteurs à agitation, on utilise le troisième réacteur pour une réaction ultérieure à une température de 80 à 100 °C, pour obtenir une solution aqueuse de bétaïne qui présente une teneur en amine tertiaire de départ encore inférieure par comparaison à la solution de bétaïne du deuxième réacteur à agitation.

**3.** Procédé selon la revendication 1, caractérisé en ce que l'on effectue la réaction dans deux réacteurs à agitation disposés en cascade, et en ce que l'on procède de façon à

a) introduire en même temps et en continu dans le premier réacteur à agitation l'amine tertiaire et l'acide ω-halogénocarboxylique en une quantité de 1 à 1,15 mole par mode d'amine tertiaire, l'hydroxyde de métal alcalin en une quantité de seulement 0,85 à 0,95 mode par mode d'acide ω-halogénocarboxylique, et l'eau en une quantité telle que la teneur en bétaïne de la solution aqueuse de bétaïne finale soit de 25 à 40 % en masse, et maintenir dans ce réacteur, à une température de 70 à 95 °C, un temps de séjour tel que le produit quittant ce réacteur et entrant dans le second réacteur à agitation contienne encore 3 à 10 % en modes d'amine tertiaire, par rapport à l'amine tertiaire introduite, et à

b) introduire en continu dans le second réacteur à agitation une nouvelle quantité d'hydroxyde de métal alcalin, qui correspond à la différence entre une quantité allant de la quantité stoechiométrique d'hydroxyde de métal alcalin à un excès de 3 % en modes et celle introduite dans le premier réacteur, et on maintient dans ce réacteur, à une température de 70 à 95 °C, un temps de séjour tel que la solution aqueuse de bétaïne quittant le réacteur contienne moins de 2 % en modes d'amine tertiaire, par rapport à d'amine tertiaire introduite.

**4.** Procédé selon la revendication 1, caractérisé en ce que l'on effectue la réaction dans trois réacteurs à agitation disposés en cascade, et en ce que l'on procède de façon à

a) introduire en même temps et en continu dans le premier réacteur à agitation l'amine tertiaire et l'acide ω-halogénocarboxylique en une quantité de 1 à 1,15 mode par mode d'amine tertiaire, l'hydroxyde de métal alcalin en une quantité de seulement 0,85 à 0,95 mode par mode d'acide ω-halogénocarboxylique, et l'eau en une quantité telle que la teneur en bétaïne de la solution aqueuse de bétaïne finale soit de 25 à 40 % en masse, et maintenir dans ce réacteur, à une température de 70 à 95 °C, un temps de séjour tel que le produit quittant ce réacteur et entrant dans le deuxième réacteur à agitation contienne encore 3 à 10 % en modes d'amine tertiaire, par rapport à l'amine tertiaire introduite, et à

b) introduire en continu dans le deuxième réacteur à agitation une nouvelle quantité d'hydroxyde de métal alcalin, qui correspond à la différence entre la quantité stoechiométrique d'hydroxyde de métal alcalin et celle introduite dans le premier réacteur, et maintenir dans ce réacteur, à une température de 70 à 95 °C, un temps de séjour tel que la solution aqueuse de bétaïne quittant ce réacteur et entrant dans le troisième réacteur contienne moins de 2 % en modes d'amine tertiaire, par rapport à l'amine tertiaire introduite, et

c) introduire en continu dans le troisième réacteur à agitation une nouvelle quantité d'hydroxyde de métal alcalin allant jusqu'à un excès stoechiométrique de 3 % en moles, et maintenir dans ce réacteur, à une température de 80 à 100 °C, un temps de séjour tel que la solution aqueuse de bétaïne quittant le réacteur contienne un pourcentage en modes d'amine tertiaire encore inférieur à celui de la solution de bétaïne entrant dans le réacteur.

**5.** Procédé selon l'une des revendications 1 à 4, caractérisé en ce que l'on maintient dans le deuxième réacteur à agitation un temps de séjour tel que la solution aqueuse de bétaïne quittant le réacteur contienne moins de 1 % en modes d'amine tertiaire par rapport à l'amine tertiaire introduite.

**6.** Procédé selon l'une des revendications 1 à 5, caractérisé en ce que l'on utilise comme amine tertiaire une amine de formule 2 dans laquelle: $R^1$ représente un reste alkyle de 8 à 18 atomes de carbone ou un reste de formule $R'CONH(CH_2)_z$-, où R' est un reste alkyle de 8 à 18 atomes de carbone et z est 2, 3 ou 4, $R^2$ représente un reste alkyle de 1 à 4 atomes de carbone et $R^3$ représente aussi un reste alkyle de 1 à 4 atomes de carbone, comme acide ω-halogénocarboxylique l'acide monochloracétique et comme hydroxyde de métal alcalin l'hydroxyde de sodium ou l'hydroxyde de potassium.